(19) Europäisches Patentamt
European Patent Office
Office européen des brevets

(11) Publication number: 0 391 124 B1

(12) # EUROPEAN PATENT SPECIFICATION

(45) Date of publication of patent specification: **21.06.95** (51) Int. Cl.6: **A61K 7/48**

(21) Application number: **90105238.1**

(22) Date of filing: **20.03.90**

(54) **Cosmetic composition of double emulsion type.**

(30) Priority: **05.04.89 JP 86091/89**
**02.02.90 JP 24044/90**

(43) Date of publication of application:
**10.10.90 Bulletin 90/41**

(45) Publication of the grant of the patent:
**21.06.95 Bulletin 95/25**

(84) Designated Contracting States:
**AT CH DE ES FR GB LI NL**

(56) References cited:
**EP-A- 0 076 146**
**EP-A- 0 154 837**
**EP-A- 0 281 394**
**FR-A- 2 268 516**
**US-A- 4 423 041**

(73) Proprietor: **KAO CORPORATION**
**14-10, Nihonbashi Kayaba-cho 1-chome**
**Chuo-ku**
**Tokyo (JP)**

(72) Inventor: **Tokimitsu, Ichiro**
**7-23-616, Shin Ogawa-cho**
**Shinjuku-ku,**
**Tokyo (JP)**
Inventor: **Kobayashi, Kumiko**
**2-7-12, Oosawa**
**Koshigaya-shi,**
**Saitama-ken (JP)**
Inventor: **Uzu, Atsushi**
**1-11-1, Yatsu-machi**
**Narashino-shi,**
**Chiba-ken (JP)**
Inventor: **Arisawa, Masatoshi**
**523-8 Koyama**
**Matsudo-shi,**
**Chiba-ken (JP)**

(74) Representative: **Patentanwälte Grünecker,**
**Kinkeldey, Stockmair & Partner**
**Maximilianstrasse 58**
**D-80538 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 391 124 B1

## Description

The present invention relates to a cosmetic composition of double emulsion type, particularly one excellent in skin-care effect.

Emulsions useful as cosmetics are classified into two groups, i.e., F/W emulsion and W/F emulsion. It is known that a W/F emulsion exhibits higher occlusiveness than that of an F/W emulsion when applied to the skin to thereby prevent the transpiration of watery vapor from the skin, thus keeping the skin moist for a longer time.

However, a W/F emulsion is inferior to an F/W emulsion in respect of feelings in use, particularly spreadability and stickiness, thus being not widespread as yet. Particularly, a W/F emulsion containing an intercellular lipid of the horny layer which is solid at ordinary temperatures or an analog thereof, which has recently been known to exhibit a high skin-care effect, has another problem in that the lipid is liable to crystallize out from the emulsion, which makes the commercialization thereof difficult.

EP-A-0281394 discloses a cosmetic composition of double emulsion type of oil-in-water-in-silicone fluid (O/W/S) comprising (a) a silicone fluid continuous phase, (b) an aqueous discontinuous phase comprising an oil-in-water (O/W) emulsion and (c) an effective dispersing amount of dimethicone copolyol for dispersing (b) in (a). The first oil phase is an oily liquid non-particulate phase.

An object of the present invention is to provide a cosmetic composition of double emulsion type which stably contains a fat which is solid at room temperatures and which exhibits high occlusiveness for the skin, thus being excellent in humectant effect, and is excellent in feelings in use.

The inventors of the present invention have extensively studied and have found that a cosmetic composition of F/W/O double emulsion type which stably contains a fat which is solid at room temperatures and which exhibits high occlusiveness for the skin and is excellent in feelings in use can be prepared by preliminarily preparing an F/W emulsion containing a large amount of a fat which is solid at room temperatures by the use of a specific emulsifying agent under specific conditions and mixing the F/W emulsion with an oil phase containing a large amount of an oil which is liquid at room temperatures by the use of a specific emulsifying agent.

The present invention has been accomplished on the basis of this finding and provides a cosmetic composition of F/W/O double emulsion type which is obtainable by the process

(A) mixing a solid fat phase consisting essentially of 1 to 30% by weight of at least one fat, which is solid at 25 °C, selected from the group consisting of cholesterol, cholesteryl isostearate, palmitic acid, stearic acid, cetyl alcohol, stearyl alcohol and amides represented by the following general formula [1];

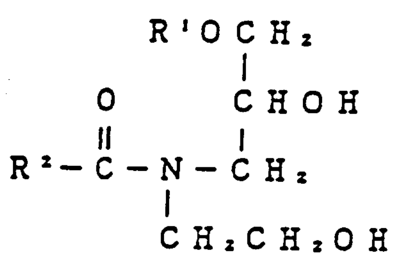

(I)

Wherein $R^1$ stands for a straight-chain or branched, saturated or unsaturated hydrocarbon group having 10 to 26 carbon atoms and $R^2$ stands for a straight-chain or branched, saturated or unsaturated hydrocarbon group having 9 to 25 carbon atoms,

and 0.01 to 5% by weight of a hydrophilic emulsifying agent, with a water phase containing water and 1 to 30% by weight of a water-soluble humectant to obtain a F/W emulsion and

(B) mixing this F/W emulsion with a continuous oil phase containing 10 to 70% by weight of an oil which is liquid at 25 °C and 0.5 to 10% by weight of a lipophilic emulsifying agent.

The cosmetic composition of double emulsion type according to the present invention stably contains a fat which is solid at room temperatures, has a high occlusive effect on the skin to be therefor excellent in humectant effect and is excellent in feelings in use.

The fat which is solid at room temperatures (hereinafter referred to merely as "solid fat") which constitutes the solid fat phase according to the present invention is one having a softening point higher than 25 °C (the determination of softening point is according to the ring and ball method, as stipulated in Japanese Standards of Cosmetic Ingredients, General Test Method 30, that a definite weight of steel ball is

put on a sample of definite thickness and heated to thereby determine the temperature when the steel ball goes down to a definite distance).

The amount of the solid fat to be used is 1 to 30% by weight, preferably 3 to 20% by weight. If the amount is less than 1% by weight, any objective skin-care effect will not be attained, while if the amount exceeds 30% by weight, the storage stability of the resulting cosmetic will be low.

The above solid fat phase contains a hydrophilic emulsifying agent which will be described below and, if necessary, other components such as liquid oil or lipophilic emulsifying agent in addition to the above solid fat.

The oil which is liquid at 25°C (hereinafter referred to merely as "liquid oil") which constitutes the continuous oil phase according to the present invention includes polysiloxanes represented by the general formula [II] to [IV] which will be described below; vegetable oils such as olive oil and jojoba oil; hydrocarbon oils such as liquid paraffin, liquid polyisobutylene and perfluoropolyether disclosed in JP-A- 234928/1986. These liquid oils may be used either alone or mixedly.

The amount of the liquid oil to be used is 10 to 70% by weight, preferably 30 to 60% by weight. If the amount is less than 10% by weight, no stable F/W/O emulsion will be formed, while if the amount exceeds 70% by weight, the resulting cosmetic will unfavorably exhibit too strong oiliness in use.

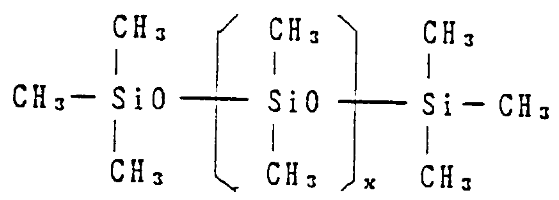
[II]

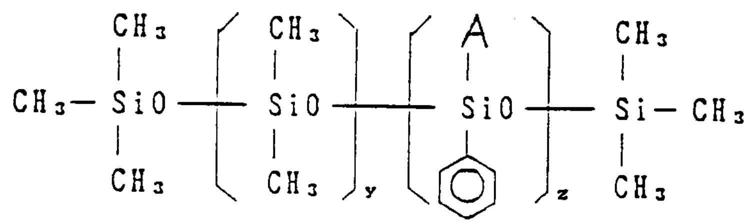
[III]

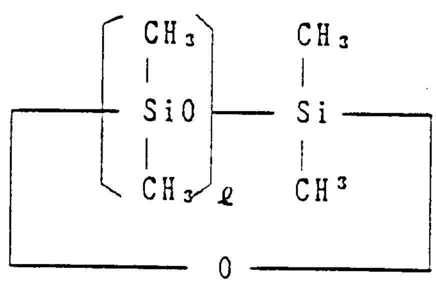
[IV]

wherein A stands for an methyl group or phenyl group; x stands for an integer of 4 to 100; z stands for an integer of 1 or above; the sum of y and z is an integer of 1 to 100 and ℓ stands for an integer of 2 to 5.

The above continuous oil phase forms an outermost continuous phase in the F/W/O emulsion according to the present invention and contains a lipophilic emulsifying agent which will be described below and, if necessary, other components in addition to the liquid oil.

The water-soluble humectant constituting the aqueous phase according to the present invention includes polyols such as glycerin, propylene glycol and sorbitol; amino acids; pyrrolidonecarboxylic acid and urea. The amount of the water-soluble humectant used is 1 to 30% by weight, preferably 2 to 20% by weight. If the amount is less than 1% by weight, any objective skin-care effect will not be attained, while if it exceeds 30% by weight, the resulting cosmetic will be problematically sticky in use.

Although the aqueous phase contains water, ethanol and other water-soluble components in addition to the above humectant, the amount of the water, etc. to be used may be suitably adjusted.

The hydrophilic and lipophilic emulsifying agents to be used in the present invention may be each any one which is conventionally used for cosmetics.

Examples of the hydrophilic emulsifying agent include nonionic surface active agents such as fatty acid esters of polyoxyethylene sorbitan and fatty acid esters of sucrose in which HLB thereof is 10 or more; and ionic surface active agents such as salts of alkyl phosphate, salts of alkyl sulfate, salts of polyoxyethylene alkylether phosphate and soap. The amount of the emulsifying agent used is 0.01 to 5% by weight, preferably 0.5 to 3% by weight. If the amount is less than 0.01% by weight, no stable emulsion will be formed, while if it exceeds 5% by weight, the resulting cosmetic will be sticky in use, thus being uncomfortable to the touch.

The above hydrophilic emulsifying agent is supposed to have the function to prevent the dissolution thereof into the continuous oil phase to thereby form a stable double emulsion.

On the other hand, examples of above lipophilic emulsifying agent include nonionic surface active agents such as fatty acid monoglyceride, fatty acid esters of sorbitan, alkyl glyceryl ether in which HLB thereof is 6 or below, phospholipid having 2 acyl groups such as lecithin and modified silicone such as dimethylpolysiloxane-polyoxyalkylene copolymers represented by the following general formula [V]:

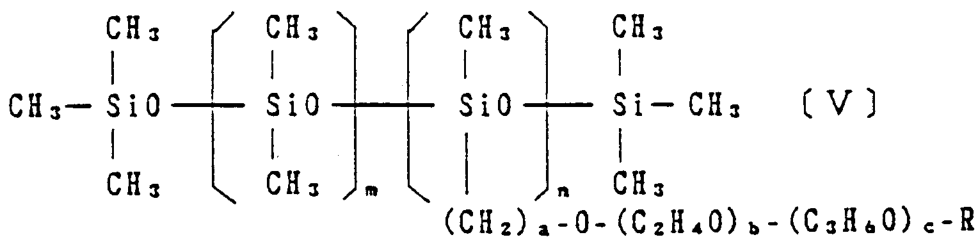

[V]

wherein a is an integer of 1 to 5; b is an integer of 7 to 40; c is an integer of 0 to 40, m is an integer of 20 to 500, n is an integer of 1 to 7 and R is a hydrogen atom or an alkyl group having 1 to 5 carbon atoms.

The amount of the above emulsifying agent to be used is 0.5 to 10% by weight, preferably 1 to 5% by weight. If the amount is less than 0.5% by weight, no stable emulsion will be formed, while if it exceeds 10% by weight, the resulting cosmetic will be sticky in use, thus being uncomfortable to the touch. It is preferable that the emulsifying agent has an HLB of 6 or below.

The F/W/O emulsion (i.e., cosmetic composition of double emulsion type) according to the present invention can be prepared according to, for example, the following procedure:

First, a solid fat phase having a prescribed composition which forms an innermost phase is heated to a temperature which is equal to the melting point of the solid fat or higher, for example, 80 °C and added under stirring to an aqueous phase having a prescribed composition which has been heated to the same temperature as that of the solid fat phase to obtain an F/W pre-emulsion. This pre-emulsion was homogenized with a homogenizer and cooled to about a room temperature to obtain an F/W emulsion. In this step, it is preferable that the ratio of the aqueous phase to the solid fat phase be between 10 : 1 and 1 : 1.

The F/W emulsion prepared above is heated again to about 60 °C and mixed with a continuous oil phase having a prescribed composition, which forms an outermost phase, to carry out the emulsification. In this step, it is preferable that the outermost oil phase have preliminarily been heated to the same temperature as that of the F/W emulsion. After the homogeneous emulsification, the obtained mixture is cooled to about a room temperature to obtain an F/W/O emulsion. In this step, it is preferable than the ratio of the F/W emulsion to the continuous oil phase be between 5 : 1 and 1 : 2, preferably between 3 : 1 and 1 : 1.

The F/W/O emulsion according to the present invention is applied by an ordinary method to various cosmetics, for example, makeup base preparations such as cream and milky lotion; makeup preparations such as foundation and hair care products such as hair tonic or hair treatment. These cosmetics may be each forced into an aerosol can together with a conventional propellant and used in the form of spray or mousse.

The F/W/O emulsion of the present invention may suitably contain other components in addition to the above essential components as far as the effect according to the present invention is not adversely affected. Examples of such components include pH adjustors, preservatives, thickening agents, perfumes and other conventional additives for cosmetics.

The present invention will now be described in more detail by referring to the following Examples.

Example 1

Milky lotions of Comparisons 1 and 2 and Inventions 1 and 2 which are each composed of an emulsion having a composition specified in Table 1 were prepared according to the preparation procedure which will be described below and examined for performance in use and humectant effect. The results are given in Table 2. The total amount of each composition listed in Table 1 is 100 parts by weight.

In Table 1,

intercellular lipid analog (4) refers to an amide represented by the general formula (I) wherein $R^1$ is $C_{16}H_{33}$ and $R^2$ is $C_{15}H_{31}$,

methylpolysiloxane (9) refers to one represented by the general formula (II) wherein x is 8,

methylpolycyclosiloxane (10) refers to one represented by the general formula (IV) wherein $\ell$ is 3,

isoparaffin (11) refers to Parleam EX (a product of Nippon Oils & Fats Co., Ltd.),

dimethylpolysiloxane-polyoxyalkylene copolymer (14) refers to one represented by the general formula (V) wherein a is 1, b is 25, c is 25, m is 400, n is 4 and R is a hydrogen atom.

Table 1

| | Component | Comparison 1 F/W emulsion | Comparison 2 W/F emulsion | Invention 1 | Invention 2 |
|---|---|---|---|---|---|
| Solid fat | (1) cholesterol | 2.0 | 2.0 | 1.0 | 1.0 |
| | (2) stearic acid | 3.0 | 3.0 | 1.5 | 1.5 |
| | (3) cetyl alcohol | 3.0 | 3.0 | 1.5 | 1.5 |
| | (4) intercellular lipid analog * | 5.0 | 5.0 | 2.5 | 2.5 |
| Hydrophilic emulsifying agent | (5) sodium polyoxyethylene (4) laurylether phosphate | 2.0 | | 1.0 | 1.0 |
| Humectant | (6) glycerin | 10.0 | 10.0 | 5.0 | 5.0 |
| | (7) propylene glycol | 3.0 | 3.0 | 1.5 | 1.5 |
| | (8) sorbitol | 1.0 | 1.0 | 0.5 | 0.5 |
| Liquid oil | (9) methylpolysiloxane * | | | 20.0 | |
| | (10) methylpolycyclosiloxane * | | | 10.0 | |
| | (11) isoparaffin | | | | 25.0 |
| | (12) olive oil | | | | 10.0 |
| Lipophilic emulsifying agent | (13) fatty acid (stearic acid) monoglyceride | | 1.0 | | 3.0 |
| | (14) dimethylpolysiloxane-polyoxyalkylene copolymer * | | | 3.0 | |
| Others | (15) methylparaben | 0.2 | 0.2 | 0.2 | 0.2 |
| | (16) perfume | 0.1 | 0.1 | 0.1 | 0.1 |
| | (17) purified water | the balance | the balance | the balance | the balance |

(Preparation procedure)

Comparison 1

Components (6) to (8) and (15) to (17) listed in Table 1 were mixed together and dissolved under heating to obtain an aqueous phase. This aqueous phase was kept at 80 °C. Separately, components (1) to

(5) listed in Table 1 were melted together under heating and gradually added to the aqueous phase. The obtained mixture was emulsified with a homogenizer and cooled to 30 ° C by the use of a heat exchanger to obtain and F/W emulsion. This emulsion was poured into a suitable bottle to obtain a milky lotion (Comparison 1).

Comparison 2

Components (1) to (4) and (13) were mixed together and melted under heating to obtain a fat phase. This fat phase was kept at 80 ° C. Separately, components (6) to (8) and (15) to (17) were heated together to 80 ° C to obtain a solution. This solution was gradually added to the above fat phase. The obtained mixture was emulsified with a homogenizer and cooled to 30 ° C by the use of a heat exchanger to obtain an W/F emulsion. This emulsion was poured into a suitable bottle to obtain a milky lotion (Comparison 2).

Invention 1

Components (6) to (8) and (15) to (17) listed in Table 1 were mixed together and dissolved under heating to obtain an aqueous phase. This aqueous phase was kept at 80 ° C. Separately, components (1) to (5) were melted together under heating to obtain a solid fat phase. The solid fat phase was gradually added to the above aqueous phase. The obtained mixture was emulsified with a homogenizer to obtain an F/W emulsion. This F/W emulsion was cooled to 30 ° C by the use of a heat exchanger, heated again to 60 ° C and gradually added to a continuous oil phase comprising components (9), (10) and (14) which had preliminarily been heated to 60 ° C. The obtained mixture was emulsified with a homogenizer and cooled to 30 ° C by the use of a heat exchanger to obtain an F/W/O emulsion. This emulsion was poured into a suitable bottle to obtain a milky lotion (Invention 1).

Invention 2

A milky lotion was prepared in a similar manner to that of Invention 1 except that the components listed in the column of Invention 2 (not 1) were used.

(Test method)

(1) Practical test (panel test)

The expert panellists practically used the milky lotions prepared above to evaluate the performance in use, i.e., spreadability, unstickiness and moisturized feeling of the skin after 3 hours from the application, according to the four criteria which will be described below. The results are given in Table 2.

: at least 8 out of the ten panellists answered "good"
: at least 6 out of the ten panellists answered "good"
: at least 4 out of the ten panellists answered "good"
: less than 4 out of the ten panellists answered "good"

(2) Determination of moisturizing effect

A predetermined amount of each sample was applied to the inside of the forearm and allowed to stand for 3 hours. The forearm was washed with warm water and kept in a thermohygrostatic room (20 ° C and 50% humidity) for one hour. the moisture content of the horny layer was determined with an impedance meter (mfd. by IBS). The average of eight measurements thus determined is shown in Table 2.

Table 2

| | | Comparison | | Invention | |
|---|---|---|---|---|---|
| | | 1 | 2 | 1 | 2 |
| Practical test | spreadability | ○ | × | ◎ | ○ |
| | unstickiness | △ | × | ◎ | ○ |
| | moisturization after 3 hours | × | ○ | ○ | ○ |
| Moisturizing effect (unit : uΩ) | | 32 | 56 | 60 | 72 |

It has been ascertained based on the results given in Table 2 that the milky lotions each composed of a double emulsion (Inventions 1 and 2) are superior to those each composed of an F/W emulsion (Comparison 1) or a W/F emulsion (Comparison 2) in feelings in use and moisturizing effect. Further, the milky lotions of Inventions 1 and 2 did not cause crystallization even after the lapse of time.

Example 2

A liquid foundation having a composition (total amount : 100 parts by weight) specified in Table 3 was prepared by the preparation procedure which will be described below (Invention 3).

In Table 3,

intercellular lipid analog (4) refers to an amide represented by the general formula (I) wherein $R^1$ is $C_{16}H_{33}$ and $R^2$ is $C_{15}H_{31}$,

fatty acid monoglyceride (11) refers to "Rheodol MS-60" (a product of Kao Corporation, fatty acid mainly consists of stearic acid),

methylpolysiloxane (8) refers to one represented by the general formula (II) wherein x is 8,

and

silicone-coated powder for cosmetic (14) is one prepared by adding 2% of methylhydrogenpolysiloxane (trade name : KF 99, a product of Shin-Etsu Chemical Co., Ltd.) to a powdery raw material comprising the following components and heating the obtained mixture:

| | |
|---|---|
| titanium dioxide | 8 (parts by weight) |
| talc | 4 |
| red oxide of iron | 1.2 |
| yellow oxide of iron | 2.6 |
| black oxide of iron | 0.2 |

(Preparation procedure)

Components (6), (7), (12) and (16) were mixed together and dissolved under heating to obtain an aqueous phase. This aqueous phase was kept at 80 °C. Separately, components (1) to (5) were melted together under heating to obtain a solid fat phase. This solid fat phase was gradually added to the above aqueous phase. The obtained mixture was emulsified with a homogenizer to obtain an F/W emulsion. This F/W emulsion was cooled to 30 °C by the use of a heat exchanger, heated again to 60 °C and gradually added to a continuous oil phase comprising components (8) to (11), (13), (14) and (15) which had preliminarily been heated to 60 °C. The obtained mixture was emulsified with a homogenizer and cooled to

30 °C by the use of a heat exchanger to obtain an F/W/O emulsion. This F/W/O emulsion was poured into a suitable bottle to obtain a liquid foundation (Invention 3).

Table 3

| | Component | Invention 3 |
|---|---|---|
| Solid fat | (1) cholesteryl isostearate | 2.0 |
| | (2) stearic acid | 1.0 |
| | (3) palmitic acid | 1.0 |
| | (4) intercellular lipid analog * | 5.0 |
| Hydrophilic emulsifying agent | (5) arginine cetylphosphate | 2.0 |
| Humectant | (6) glycerin | 3.0 |
| | (7) sorbitol | 2.0 |
| Liquid oil | (8) methylpolysiloxane * | 12.0 |
| | (9) olive oil | 10.0 |
| Lipophilic emulsifying agent | (10) lecithin | 1.0 |
| | (11) fatty acid monoglyceride * | 3.0 |
| Others | (12) methylparaben | 0.2 |
| | (13) butylparaben | 0.2 |
| | (14) silicone-coated powder for cosmetic * | 16.0 |
| | (15) perfume | 0.1 |
| | (16) purified water | the balance |

Example 3

A skin care lotion having a composition (total amount : 100 parts by weight) specified in Table 4 was prepared by the preparation procedure which will be described below (Invention 4).

Each component of (1) to (8), each component of (9), (14), (15) and (17) and each component of (10) to (13) and (16) comprise the solid fat phase, the aqueous phase and the continuous oil phase, respectively.

In Table 4, an amide represented by the general formula (I) wherein $R^1$ is $C_{16}H_{33}$ and $R^2$ is $C_{15}H_{31}$ is employed as intercellular lipid analog (1).

In Table 4,
methylpolysiloxane (11) refers to one represented by the general formula (II) wherein x is 20, methylpolycyclosiloxane (10) refers to one represented by the general formula (IV) wherein $\ell$ is 3,
and
dimethylpolysiloxane-polyoxyalkylene copolymer (12) refers to one represented by the general formula (V) wherein a is 1, b is 25, c is 25, m is 400 and R is a hydrogen atom.

(Preparation procedure)

Components (9), (14), (15) and (17) listed in Table 4 were mixed together and dissolved under heating to obtain an aqueous phase. This aqueous phase was kept at 80 °C. Separately, components (1) to (8) were melted together under heating to obtain a solid fat phase. This solid fat phase was gradually added to the above aqueous phase. The obtained mixture was emulsified with a homogenizer to obtain an F/W emulsion. This F/W emulsion was cooled to 30 °C by the use of a heat exchanger, heated again to 60 °C and gradually added to a continuous oil phase comprising components (10) to (13) and (16) which had been preliminarily heated to 80 °C. The obtained mixture was emulsified with a homogenizer and cooled to 30 °C by the use of a heat exchanger to obtain an F/W/O emulsion. This F/W/O emulsion was filled into a suitable

jar to obtain a skin care cream (Invention 4).

Table 4

| | Component | Invention 4 |
|---|---|---|
| Solid fat | (1) intercellular lipid analog * | 1.50 |
| | (2) cholesterol | 0.50 |
| | (3) cholesteryl isostearate | 1.00 |
| | (4) stearic acid | 0.50 |
| | (5) cetyl alcohol | 0.50 |
| Liquid oil | (6) squalane | 4.00 |
| | (7) isostearic acid diglyceride | 0.25 |
| Hydrophilic emulsifying agent | (8) arginine 2-hexyldecyl phosphate | 0.15 |
| Humectant | (9) glycerin | 2.85 |
| Liquid oil | (10) methylpolycyclosiloxane * | 30.00 |
| | (11) methylpolysiloxane * | 10.00 |
| Lipophilic emulsifying agent | (12) dimethylpolysiloxane-polyoxyalkylene copolymer * | 5.00 |
| | (13) α-monoisostearyl glyceryl ether | 1.00 |
| Others | (14) methylparaben | 0.30 |
| | (15) ethanol | 3.00 |
| | (16) polystyrene powder | 1.00 |
| | (17) purified water | the balance |

## Claims

**1.** A cosmetic composition of F/W/O double emulsion obtainable by the process

(A) mixing a solid fat phase consisting essentially of 1 to 30% by weight of at least one fat, which is solid at 25 °C, selected from the group consisting of cholesterol, cholesteryl isostearate, palmitic acid, stearic acid, cetyl alcohol, stearyl alcohol and amides represented by the following general formula [1];

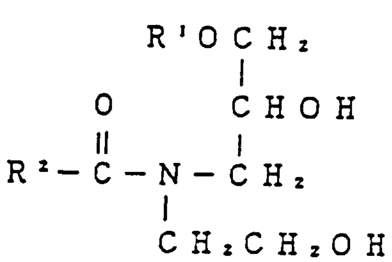

[ I ]

Wherein $R^1$ stands for a straight-chain or branched, saturated or unsaturated hydrocarbon group having 10 to 26 carbon atoms and $R^2$ stands for a straight-chain or branched, saturated or unsaturated hydrocarbon group having 9 to 25 carbon atoms,

and 0.01 to 5% by weight of a hydrophilic emulsifying agent, with a water phase containing water and 1 to 30% by weight of a water-soluble humectant to obtain a F/W emulsion and

(B) mixing this F/W emulsion with a continuous oil phase containing 10 to 70% by weight of an oil which is liquid at 25 °C and 0.5 to 10% by weight of a lipophilic emulsifying agent.

2. A cosmetic composition of F/W/O double emulsion type as set forth in claim 1, wherein the ratio of the aqueous phase to the solid fat phase is between 10 : 1 and 1 : 1.

3. A cosmetic composition of F/W/O double emulsion type as set forth in claim 1, wherein the ratio of the F/W emulsion to the continuous oil phase is between 5 : 1 and 1 : 2.

4. A cosmetic composition of F/W/O double emulsion type as set forth in claim 1, wherein the hydrophilic emulsifying agent is one or more hydrophilic emulsifying agents selected from the group consisting of fatty acid esters of polyoxyethylene sorbitan and fatty acid esters of sucrose in which HLB thereof is 10 or more; and ionic surface active agents such as salts of alkyl phosphate, salts of alkyl sulfate, salts of polyoxyethylene alkylether phosphate and soap.

5. A cosmetic composition of F/W/O double emulsion type as set forth in claim 1, wherein the water-soluble humectant is one or more water-soluble humectants selected from the group consisting of polyols such as glycerin, propylene glycol and sorbitol; amino acids; pyrrolidonecarboxylic acid and urea.

6. A cosmetic composition of F/W/O double emulsion type as set forth in claim 1, wherein the liquid oil is one or more liquid oils selected from the group consisting of polysiloxanes represented by the general formula [II] to [IV] which will be described below; vegetable oils such as olive oil and jojoba oil; hydrocarbon oils such as liquid paraffin, liquid polyisobutylene and perfluoro polyether.

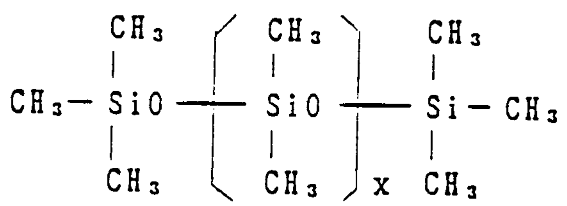

[ II ]

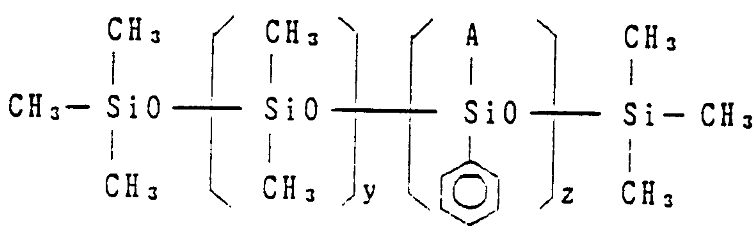

[ III ]

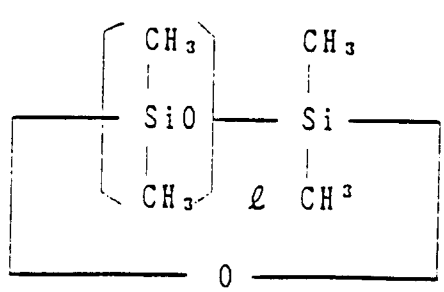

[ IV ]

wherein A stands for an methyl group or phenyl group; x stands for an integer of 4 to 100; z stands for an integer of 1 or above; the sum of y and z is an integer of 1 to 100 and $\ell$ stands for an integer of 2 to 5.

7. A cosmetic composition of F/W/O double emulsion type as set forth in claim 1, wherein the lipophilic emulsifying agent is one or more lipophilic emulsifying agents selected from the group consisting of nonionic surface active agents such as fatty acid monoglyceride, fatty acid esters of sorbitan, alkyl glyceryl ether in which HLB thereof is 6 or below, phospholipid having 2 acyl groups such as lecithin and modified silicone such as dimethylpolysiloxane-polyoxyalkylene copolymers represented by the

following general formula [V]:

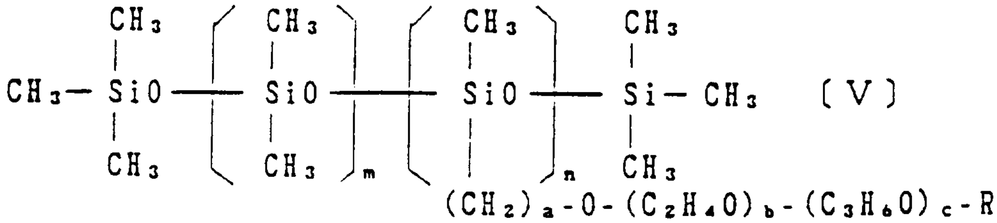

[V]

wherein a is an integer of 1 to 5; b is an integer of 7 to 40; c is an integer of 0 to 40, m is an integer of 20 to 500, n is an integer of 1 to 7 and R is a hydrogen atom or an alkyl group having 1 to 5 carbon atoms.

**Patentansprüche**

1. Kosmetische F/W/O-Doppelemulsionszusammensetzung, erhältlich durch das Verfahren
(A) Mischen einer festen Fettphase, die im wesentlichen aus 1 bis 30 Gew.-% mindestens eines Fettes, das bei 25 °C fest ist, ausgewählt aus der Gruppe, bestehend aus Cholesterin, Cholesterinisostearat, Palmitinsäure, Stearinsäure, Cetylalkohol, Stearylalkohol und Amiden, dargestellt durch die folgende allgemeine Formel [1]:

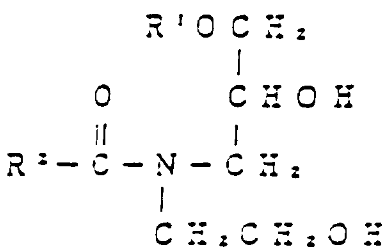

(I)

worin $R^1$ eine geradkettige oder verzweigte, gesättigte oder ungesättigte Kohlenwasserstoffgruppe mit 10 bis 26 Kohlenstoffatomen bedeutet, und $R^2$ eine geradkettige oder verzweigte, gesättigte oder ungesättigte Kohlenwasserstoffgruppe mit 9 bis 25 Kohlenstoffatomen bedeutet,
und 0,01 bis 5 Gew.-% eines hydrophilen Emulgators besteht, mit einer Wasserphase, die Wasser und 1 bis 30 Gew.-% eines wasserlöslichen Feuchthaltemittels enthält, um eine F/W-Emulsion zu erhalten, und
(B) Mischen dieser F/W-Emulsion mit einer kontinuierlichen Ölphase, die 10 bis 70 Gew.-% eines Öls, das bei 25 °C flüssig ist, und 0,5 bis 10 Gew.-% eines lipophilen Emulgators enthält.

2. Kosmetische F/W/O-Doppelemulsionszusammensetzung nach Anspruch 1, worin das Verhältnis der wäßrigen Phase zur festen Fettphase zwischen 10:1 und 1:1 liegt.

3. Kosmetische F/W/O-Doppelemulsionszusammensetzung nach Anspruch 1, worin das Verhältnis der F/W-Emulsion zur kontinuierlichen Ölphase zwischen 5:1 und 1:2 liegt.

4. Kosmetische F/W/O-Doppelemulsionszusammensetzung nach Anspruch 1, worin der hydrophile Emulgator aus einem oder mehreren hydrophilen Emulgatoren besteht, ausgewählt aus der Gruppe, bestehend aus Fettsäureestern von Polyoxyethylensorbitan und Fettsäureestern von Sucrose, deren HLB-Wert 10 oder mehr beträgt; und ionischen oberflächenaktiven Mitteln, wie z.B. Salzen von Alkylphosphat, Salzen von Alkylsulfat, Salzen von Polyoxyethylenalkyletherphosphat und Seife.

5. Kosmetische F/W/O-Doppelemulsionszusammensetzung nach Anspruch 1, worin das wasserlösliche Feuchthaltemittel aus einem oder mehreren wasserlöslichen Feuchthaltemitteln besteht, ausgewählt aus

der Gruppe, bestehend aus Polyolen, wie z.B. Glycerin, Propylenglykol und Sorbitol; Aminosäuren; Pyrrolidoncarbonsäure und Harnstoff.

6. Kosmetische F/W/O-Doppelemulsionszusammensetzung nach Anspruch 1, worin das flüssige Öl aus einem oder mehreren flüssigen Ölen besteht, ausgewählt aus der Gruppe, bestehend aus Polysiloxanen, dargestellt durch die allgemeinen Formeln [II] bis [IV], die unterhalb beschrieben werden; pflanzlichen Ölen, wie z.B. Olivenöl und Jojobaöl; Kohlenwasserstoffölen, wie z.B. flüssigem Paraffin, flüssigem Polyisobutylen und Perfluorpolyether;

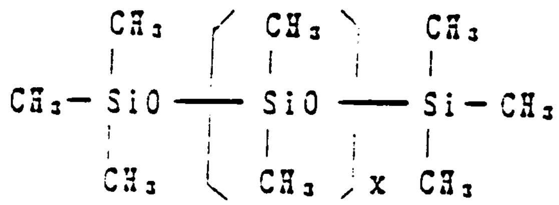

[II]

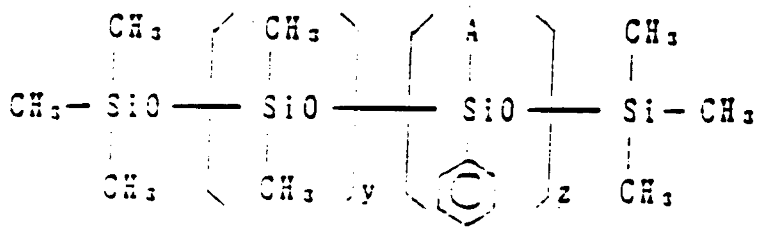

[III]

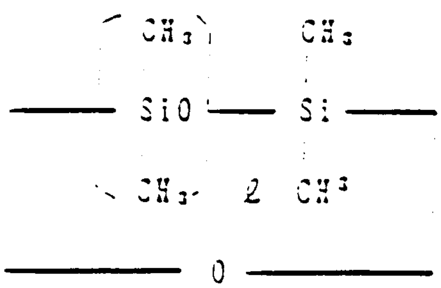

[IV]

worin A eine Methylgruppe oder Phenylgruppe bedeutet; x bedeutet eine ganze Zahl von 4 bis 100; z bedeuted eine ganze Zahl von 1 oder mehr; die Summe von y und z ist eine ganze Zahl von 1 bis 100 und ℓ bedeutet eine ganze Zahl von 2 bis 5.

7. Kosmetische F/W/O-Doppelemulsionszusammensetzung nach Anspruch 1, worin der lipophile Emulgator aus einem oder mehreren lipophilen Emulgatoren besteht, ausgewählt aus der Gruppe, bestehend aus nichtionischen oberflächenaktiven Mitteln, wie z.B. Fettsäuremonoglycerid, Fettsäureestern von Sorbitan, Alkylglycerylether, deren HLB-Wert 6 oder weniger beträgt, Phospholipid mit 2 Acylgruppen, wie z.B. Lecithin und modifiziertem Silikon, wie z.B. Dimethylpolysiloxan-Polyoxyalkylen-Copolymere, dargestellt durch die folgende allgemeine Formel [V]:

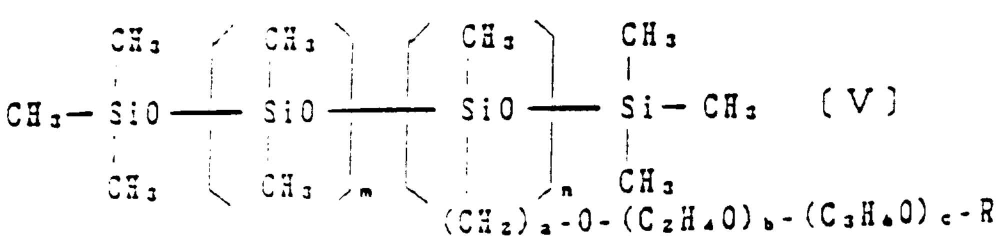

[V]

worin a eine ganze Zahl von 1 bis 5 ist; b ist eine ganze Zahl von 7 bis 40; c ist eine ganze Zahl von 0 bis 40; m ist eine ganze Zahl von 20 bis 500; n ist eine ganze Zahl von 1 bis 7; und R ist ein Wasserstoffatom oder eine Alkylgruppe mit 1 bis 5 Kohlenstoffatomen.

## Revendications

1. Composition cosmétique d'émulsion double matière grasse/eau/huile que l'on peut obtenir par le procédé consistant à

   (A) mélanger une phase grasse solide constituée essentiellement de 1 à 30% en poids d'au moins une matière grasse, qui est solide à 25°C, choisie dans le groupe constitué par le cholestérol, l'isostéarate de cholestéryle, l'acide palmitique, l'acide stéarique, l'alcool cétylique, l'alcool stéarylique et les amides représentés par la formule générale [I]:

   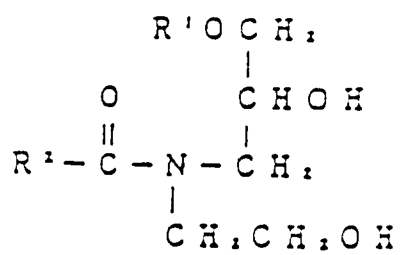

   (I)

   dans laquelle $R^1$ représente un groupe hydrocarboné saturé ou insaturé, à chaîne droite ou ramifiée, ayant de 10 à 26 atomes de carbone et $R^2$ représente un groupe hydrocarboné saturé ou insaturé, à chaîne droite ou ramifiée, ayant de 9 à 25 atomes de carbone,

   et de 0,01 à 5% en poids d'un agent émulsifiant hydrophile, avec une phase aqueuse contenant de l'eau et de 1 à 30% en poids d'un humectant soluble dans l'eau pour obtenir une émulsion matière grasse/eau et

   (B) mélanger cette émulsion matière grasse/eau avec une phase huileuse continue contenant de 10 à 70% en poids d'une huile qui est liquide à 25°C et de 0,5 à 10% en poids d'un agent émulsifiant lipophile.

2. Composition cosmétique de type émulsion double matière grasse/eau/huile selon la revendication 1 dans laquelle le rapport de la phase aqueuse à la phase grasse solide est compris entre 10:1 et 1:1.

3. Composition cosmétique de type émulsion double matière grasse/eau/huile selon la revendication 1 dans laquelle le rapport de l'émulsion matière grasse/eau à la phase huileuse continue est compris entre 5:1 et 1:2.

4. Composition cosmétique de type émulsion double matière grasse/eau/huile selon la revendication 1 dans laquelle l'agent émulsifiant hydrophile est un ou plusieurs agents émulsifiants hydrophiles choisis dans le groupe constitué par les esters d'acides gras de polyoxyéthylène sorbitane et les esters d'acides gras de saccharose dont l'équilibre hydrophile-lipophile est égal ou supérieur à 10; et les agents tensio-actifs ioniques comme les sels d'alkyl-phosphate, les sels d'alkyl-sulfate, les sels de polyoxyéthylène-alkyléther-phosphate et le savon.

5. Composition cosmétique de type émulsion double matière grasse/eau/huile selon la revendication 1, dans laquelle l'humectant soluble dans l'eau est un ou plusieurs humectants choisis dans le groupe constitué par les polyols comme la glycérine, le propylène glycol et le sorbitol; les acides aminés; l'acide pyrrolidone-carboxylique et l'urée.

6. Composition cosmétique de type émulsion double matière grasse/eau/huile selon la revendication 1 dans laquelle l'huile liquide est une ou plusieurs huiles liquides choisies dans le groupe constitué par les polysiloxanes représentés par les formules générales [II] à [IV] qui seront décrites ci-dessous; les huiles végétales comme l'huile d'olive et l'huile de jujube; les huiles hydrocarbonées comme la paraffine liquide, le polyisobutylène liquide et le perfluoro-polyéther.

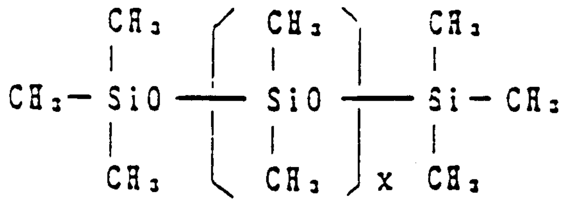

( II )

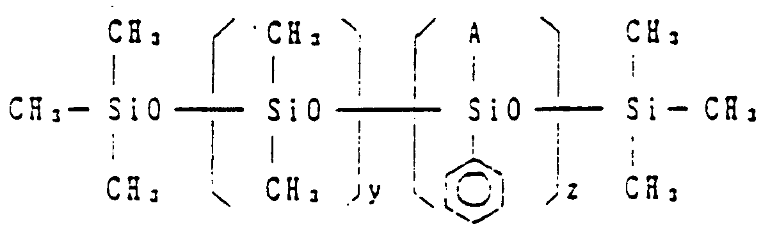

( III )

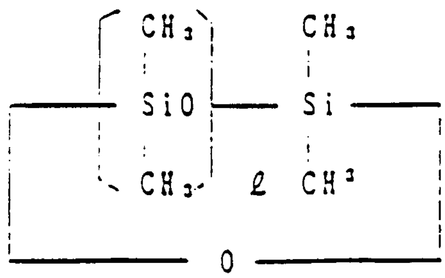

( IV )

Où A représente un groupe méthyle ou un groupe phényle; x représente un nombre entier valant de 4 à 100; z représente un nombre entier égal ou supérieur à 1; la somme de y et z est un nombre entier valant de 1 à 100 et 1 représente un nombre entier valant de 2 à 5.

**7.** Composition cosmétique de type émulsion double matière grasse/eau/huile selon la revendication 1, dans laquelle l'agent émulsifiant lipophile est un ou plusieurs agents émulsifiants lipophiles choisis dans le groupe constitué par les agents tensio-actifs non ioniques comme le monoglycéride d'acide gras, les esters d'acide gras de sorbitane, les alkyl-glycéryl-éthers dont l'équilibre hydrophile-lipophile est égal ou inférieur à 6, les phospholipides ayant 2 groupes acyle comme la lécithine et les silicones modifiées comme les copolymères diméthylpolysiloxane-polyoxyalkylène représentés par la formule générale [V] ci-dessous:

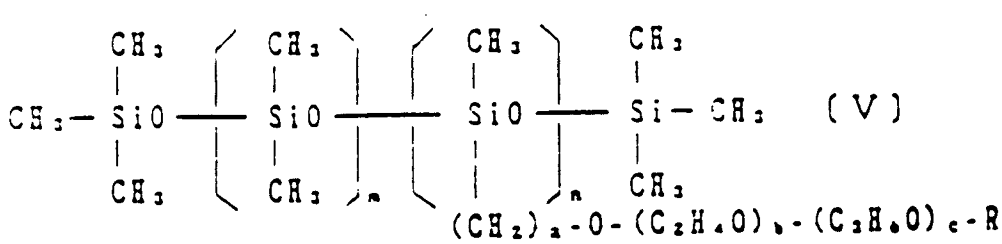

( V )

dans laquelle a est un nombre entier valant de 1 à 5; b est un nombre entier valant de 7 à 40; c est un nombre entier valant de 0 à 40, m est un nombre entier valant de 20 à 500, n est un nombre entier valant de 1 à 7 et R est un atome d'hydrogène ou un groupe alkyle ayant de 1 à 5 atomes de carbone.